# EUROPÄISCHE PATENTSCHRIFT

(11) **EP 2 470 888 B1**
(45) Veröffentlichungstag und Bekanntmachung des Hinweises auf die Patenterteilung: **10.10.2018**
(21) Anmeldenummer: 10711902.6
(22) Anmeldetag: 31.03.2010
(51) Int. Cl.: G01N 21/11, G01N 21/85, B01L 3/00, F04B 43/08, F04B 43/12, G01N 35/10, G01N 21/03, G01N 21/05, G01N 21/78, G01N 21/84, G01N 33/18, G01N 1/40

(54) **PNEUMATISCH ANGETRIEBENE MEHRKAMMER-PERISTALTIKPUMPE FÜR EIN WASSERANALYSEGERÄT**
PNEUMATICALLY ACTUATED MULTI-CHAMBER PERISTALTIC PUMP FOR A WATER ANALYSIS DEVICE
POMPE PERISTALTIQUE A MULTIPLES CHAMBRES ACTIONNE PNEUMATIQUEMENT POUR UN DISPOSITIF D'ANALYSE D'EAU

(30) Priorität: 25.08.2009 EP 09168536
(43) Veröffentlichungstag der Anmeldung: 04.07.2012
(73) Patentinhaber: Hach Lange GmbH, 14163 Berlin (DE)
(72) Erfinder: FARJAM, Aria, 40223 Düsseldorf (DE); UTHEMANN, Rolf, 51373 Leverkusen (DE); BERGGOLD, Kai, 50823 Köln (DE); LUNDGREEN, Ulrich, 33330 Gütersloh (DE); DE HEIJ, Bas, 41542 Dormagen (DE)
(74) Vertreter: Patentanwälte ter Smitten Eberlein-Van Hoof Rütten Partnerschaftsgesellschaft mbB
(86) Internationale Anmeldenummer: PCT/EP2010/054333
(87) Internationale Veröffentlichungsnummer: WO 2011/023420

(56) Entgegenhaltungen:
- EP-A2- 1 353 069
- DE-A1- 2 639 992
- US-A- 4 583 920
- US-A- 5 593 290
- US-A1- 2004 013 536
- WANG ET AL: "Automatic bio-sampling chips integrated with micro-pumps and micro-valves for disease detection" BIOSENSORS AND BIOELECTRONICS, ELSEVIER BV, NL LNKD- DOI:10.1016/J.BIOS.2004.11.004, Bd. 21, Nr. 3, 15. September 2005 (2005-09-15), Seiten 419-425, XP005001255 ISSN: 0956-5663

## Beschreibung

Gegenstand der Erfindung ist eine pneumatisch betätigte Mehrkammer-Peristaltikpumpe für ein Wasseranalysegerät.

In einem Wasseranalysegerät werden Flüssigkeiten, beispielsweise eine Wasserprobe, ein flüssiges Dialysat, ein flüssiges Analyt, eine Spülflüssigkeit etc. durch Pumpen transportiert. Wenn das Analysegerät mikrofluidisch aufgebaut ist, sind als Pumpentyp insbesondere Peristaltikpumpen geeignet. Diese weisen jeweils mehrere, bevorzugt drei, Pumpenkammern auf, die sukzessive gefüllt und geleert werden, um auf diese Weise einen unidirektionalen peristaltischen Pumpvorgang zu erzeugen.
Aus US 5 593 290 A ist eine mikrofluidische Mehrkammer-Peristaltikpumpe bekannt, die drei Pumpenkammern aufweist, die auf der proximalen Seite einer Grundplatte ausgebildet sind und jeweils durch eine elastische Pumpenmembrane verschlossen sind. Die einander benachbarten Pumpkammern sind jeweils durch einen Verbindungskanal miteinander verbunden, der in der Grundplatte in Form einer proximal offenen Nut ausgebildet ist, die durch eine Zwischenplatte verschlossen ist. Die Zwischenplatte weist im Bereich der Pumpenkammern entsprechende durchgehende Öffnungen auf. Auf der proximalen Seite der Zwischenplatte ist die elastische Pumpenmembrane angeordnet, auf der wiederum eine Deckplatte fixiert ist, die für jede Pumpkammer einen pneumatischen Aktuatorkanal aufweist.

Die Herstellung dieser Peristaltikpumpe ist äußerst anspruchsvoll, da die Grundplatte und die Zwischenplatte auf weniger als 0,1 mm genau miteinander ausgerichtet werden müssen. Um ein jeweils möglichst gleiches Gesamtvolumen der drei Pumpkammern sicherzustellen, wird die Zwischenplatte von einer dünnen Folie gebildet. Es ist technisch anspruchsvoll, in der dünnen Folie die durchgehenden Öffnungen exakt herzustellen.
In DE 2639992 ist eine Infusionspumpe in Form einer Drei-Kammer-Peristaltikpumpe offenbart.
Aus EP 1353069 A2 ist eine Pumpe mit einer Pumpkammer und zwei Ventilen bekannt. Die Ventilkammern liegen auf der der Pumpkammer gegenüberliegenden Seite der Grundplatte

Aufgabe der Erfindung ist es, eine einfach herstellbare pneumatisch betätigte Mehrkammer-Peristaltikpumpe für ein Wasseranalysegerät zu schaffen.

Diese Aufgabe wird erfindungsgemäß gelöst durch eine pneumatisch betätigte Mehrkammer-Peristaltikpumpe für ein Wasseranalysegerät mit den Merkmalen des Patentanspruches 1.

In der erfindungsgemäßen pneumatisch betätigten Mehrkammer-Peristaltikpumpe ist der Verbindungskanal zwischen zwei Pumpenkammern auf der distalen Seite der Grundplatte als Nut ausgebildet. Im Bereich der Verbindungskanal-Nut ist auf der distalen Seite der Grundplatte eine separate Nutabdeckung vorgesehen, die die distale Öffnungsseite der Verbindungskanal-Nut verschließt. Die Nut ist also nunmehr auf der den Pumpenkammern abgewandten Seite der Grundplatte vorgesehen. Die Nut ist durch eine einfache Nutabdeckung verschlossen. Eine Zwischenplatte, die Ausnehmungen oder Öffnungen enthält, wird nicht mehr benötigt. Die Nutabdeckung kann beispielsweise ein großflächiger Körper sein, der die gesamte distale Seite der Grundplatte bedeckt. Eine exakte Positionierung der Nutabdeckung ist nicht erforderlich, was den Zusammenbau erheblich vereinfacht.

Die Pumpenkammern sind durch eine einzige Pumpenmembran verschlossen, können aber selbstverständlich auch durch separate Pumpenmembranen verschlossen sein.

Die Nutabdeckung ist eine biegsame Abdeckfolie. Die Abdeckfolie ist begrenzt flexibel, so dass sie sich an Unebenheiten auf der distalen Seite der Grundplatte anpassen kann. Hierdurch werden Undichtigkeiten durch Unebenheiten vermieden, wie sie bei einer steifen Nutabdeckung nahezu unvermeidlich wären. Durch die Verwendung einer flexiblen Abdeckfolie für die Nutabdeckung kann auf der distalen Seite der Grundplatte auf eine hohe Planität verzichtet werden.

Gemäß einer bevorzugten Ausgestaltung ist die Grundplatte ein Kunststoff-Spritzgussteil, das sowohl alle Pumpenkammern als auch die als Nuten ausgebildeten Verbindungskanäle aufweist. Da die Grundplatte ausschließlich Ausnehmungen auf der distalen und der proximalen Seite der Grundplatte aufweist, ist das Entformen problemlos möglich.

Gemäß einer bevorzugten Ausgestaltung ist die Abdeckfolie transparent ausgebildet. Die Abdeckfolie ist in Bezug auf Laserstrahlung transparent, die zum Verschweißen der Abdeckfolie mit der Grundplatte verwendet wird. Die Transparenz der Abdeckfolie erlaubt es also, die Abdeckfolie mit der Grundplatte durch Laserschweißen zu verschweißen. Hierdurch wird ein zuverlässig flüssigkeitsdichtes Fixieren der Abdeckfolie durch ein automatisiertes und damit preiswertes Herstellungsverfahren ermöglicht.

Gemäß einer bevorzugten Ausgestaltung bestehen die Grundplatte und die Abdeckfolie aus dem gleichen Kunststoff, so dass sie die gleiche Schmelztemperatur aufweisen. Hierdurch wird das Verschweißen der Abdeckfolie mit der Grundplatte durch Laserschweißen vereinfacht.

Vorzugsweise sind zwischen dem Verbindungskanal auf der distalen Seite der Grundplatte und den Pumpkammern jeweils Vertikalkanäle vorgesehen, die senkrecht zu dem Verbindungskanal stehen und die Pumpkammern jeweils mit dem Verbindungskanal verbinden. Da die Vertikalkanäle die gleiche Entformungsrichtung aufweisen, wie die Pumpkammern auf der proximalen Seite und die Verbindungskanal-Nut auf der distalen Seite der Grundplatte, ist die Grundplatte problemlos Kunststoff-Spritzguss herstellbar.

Gemäß einer bevorzugten Ausgestaltung ist die Peristaltikpumpe eine mikrofluidische Peristaltikpumpe, wobei das Pumpvolumen einer Pumpkammer kleiner als 10 µl ist. Insbesondere bei einer mikrofluidische Peristaltikpumpe ist die Einhaltung der zulässigen Toleranzen technisch sehr anspruchsvoll, so dass die vorliegende Erfindung insbesondere bei einer mikrofluidischen Peristaltikpumpe zu besonderen Vereinfachungen bei der Herstellung eines Wasseranalysegerätes mit einer derartigen Peristaltikpumpe führen.

Vorzugsweise besteht das Wasseranalysegerät aus einem alle elektrischen Bauteile aufweisenden Basismodul und einem austauschbaren Kartuschenmodul, das die gesamte Fluidik einschließlich der Peristaltikpumpe aufweist. Das Kartuschenmodul kann ein Wegwerfartikel sein, der nach Erschöpfung oder Defekt ausgetauscht und weggeworfen wird.

Im Folgenden wird unter Bezugnahme auf die Zeichnungen ein Ausführungsbeispiel der Erfindung näher erläutert.

Es zeigen:
- Figur 1: eine schematische Darstellung eines Wasseranalysegerätes, bestehend aus einem Basismodul und einem austauschbaren Kartuschenmodul, in dem eine pneumatisch betätigte Mehrkammer-Peristaltikpumpe angeordnet ist, und
- Figur 2: die Peristaltikpumpe der Figur 1 im Längsschnitt.

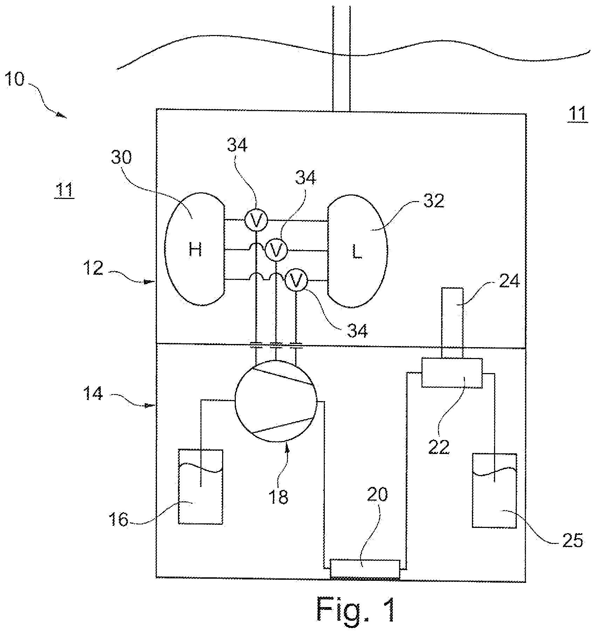

In Figur 1 ist in einer schematischen Übersicht ein Prozess-Wasseranalysegerät 10 dargestellt, das der quasi-kontinuierlichen quantitativen Bestimmung eines Analyts in Wasser 11 dient. Das Wasseranalysegerät 10 ist vorliegend vollständig in das zu untersuchende Wasser 11 eingetaucht, ist also als so genannte Tauchsonde ausgebildet, Das Wasseranalysegerät 10 ist modular aufgebaut, und besteht aus einem Basismodul 12, das alle elektrischen Komponenten aufweist, und einem austauschbaren Kartuschenmodul 14, das die gesamte Fluidik aufweist, die vorliegend mikrofluidisch ausgebildet ist. Das Kartuschenmodul 14 wird nur einmal verwendet, und wird nach Erschöpfung oder bei einem Defekt ausgetauscht und entsorgt.

Das Kartuschenmodul 14 weist einen Trägerflüssigkeitstank 16 auf, der über eine Flüssigkeitsleitung mit dem Pumpeneinlass einer pneumatisch angetriebenen Mehrkammer-Peristaltikpumpe 18 verbunden ist. Der Pumpenauslass der Peristaltikpumpe 18 ist durch eine Flüssigkeitsleitung mit einem Dialyseelement 20 verbunden, in dem das Analyt aus dem Wasser 11 in die Trägerflüssigkeit wandert.

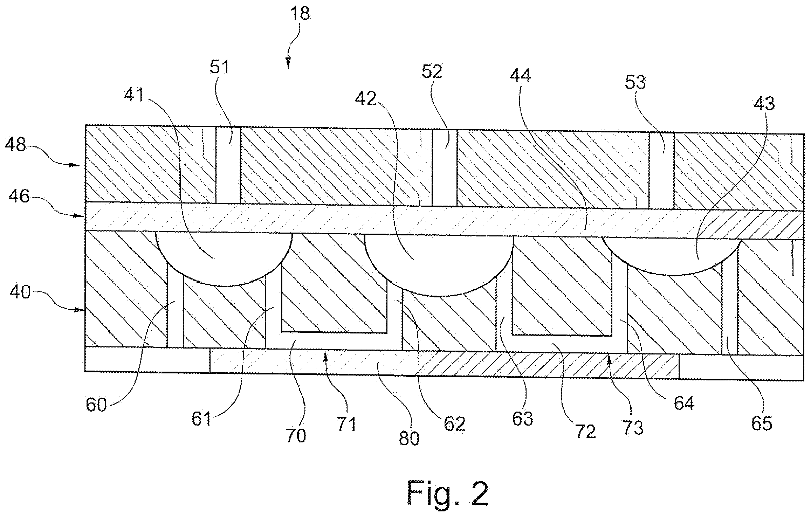

Das Dialyseelement 20 ist über eine Flüssigkeitsleitung mit einer Messstrecke 22 verbunden, wobei im Verlauf dieser Flüssigkeitsleitung ein Reagenz eingeleitet wird, das mit dem zu bestimmenden Analyt beispielsweise farbverändernd reagiert. Der Messstrecke 22 ist ein basismodulseitiges Analysegerät 24 zugeordnet, das beispielsweise als Fotometer ausgebildet sein kann und die Extinktion der Flüssigkeit in der Messstrecke 22 bestimmt. Anschließend wird die Flüssigkeit in einen Abfalltank 25 gepumpt.
Der kartuschenmodulseitige Teil der Peristaltikpumpe 18 besteht aus einer Pumpmimik, deren Aktuatorik in dem Basisgerät 12 angeordnet ist. Die Aktuatorik besteht aus einem Überdruckspeicher 30 und einem Unterdruckspeicher 32, einer nicht dargestellten Pneumatikpumpe, die mit dem Überdruckspeicher 30 und dem Unterdruckspeicher 32 verbunden ist und den erforderlichen Überdruck beziehungsweise Unterdruck erzeugt, und drei elektrisch geschalteten Umschaltventilen 34, die entweder den Überdruckspeicher 30 oder den Unterdruckspeicher 32 mit der zugeordneten Pumpenmembran der Peristaltikpumpe 18 verbinden.
Die Peristaltikpumpe 18 ist in Figur 2 im Detail dargestellt. Die Peristaltikpumpe 18 weist eine Kunststoff-Grundplatte 40 auf, die auf ihrer proximalen Seite drei Pumpkammern 41,42,43 als sphärische Ausnehmungen aufweist, die proximal eine Öffnung aufweisen. Auf der proximalen Seite 44 der Grundplatte 40 ist eine flüssigkeitsdichte und elastische Pumpenmembrane 46 angeordnet, die die Öffnungen der drei Pumpkammern 41,42,43 verschließt. Die Pumpenmembrane 46 besteht aus einem flourierten oder teilflourierten Elastomer, beispielsweise aus einem unter der Bezeichnung Viton® bekannten Material, und hat eine Stärke von 0,1 bis 0,4 mm. Die Pumpkammern 21, 42, 43 können alternativ auch durch jeweils eine separate Einzel-Pumpenmembran verschlossen sein. Diese kann aus einer kreisförmigen Membran mit einem randseitigen Ringwulst gebildet sein.

Proximal der Pumpenmembrane 46 ist eine Deckplatte 48 auf die Pumpenmembrane 46 aufgebracht, wobei die Deckplatte 48 drei pneumatische Aktuatorkanäle 51,52,53 aufweist, die ungefähr senkrecht zur Grundebene der Grundplatte 40 stehen, jeweils mit der betreffenden Pumpenkammer 41,42,43 ausgerichtet sind und ungefähr in der Mitte der Pumpenkammer-Öffnungen enden.

Von jeder Pumpenkammer 41,42,43 verlaufen jeweils zwei Vertikalkanäle 60 -65 quer zur Grundebene der Grundplatte 40 bis zur distalen Seite der Grundplatte 40. Jede Pumpenkammer 41-43 weist jeweils einen Einlass-Vertikalkanal 60,62,64 und einen Auslass-Vertikalkanal 61,63,65 auf. Der Auslass-Vertikalkanal 61 der ersten Pumpenkammer 41 ist durch einen horizontalen Verbindungskanal 71 mit dem Einlass-Vertikalkanal 62 der zweiten Pumpenkammer 42 verbunden. Der Auslass-Vertikalkanal 63 der zweiten Pumpenkammer 42 ist durch einen weiteren horizontalen Verbindungskanal 73 mit dem Einlass-Vertikalkanal 64 der dritten Pumpenkammer 42 verbunden. Die beiden Verbindungskanäle 71,73 sind jeweils als distal offene Nut 70,72 auf der distalen Seite der Grundplatte 40 ausgebildet. Die distale Seite der Grundplatte 40 ist im Bereich der Verbindungskanäle 71,73 bedeckt durch eine separate Nutabdeckung 80, die von einer biegsamen transparenten Abdeckfolie 80 gebildet wird, und die beiden Verbindungskanal-Nuten 70,72 distal verschließt.

Die Grundplatte 40 und die Abdeckfolie 80 bestehend aus demselben Kunststoff, so dass sie den gleichen Schmelzpunkt aufweisen, und sind durch Laserschweißen oder Ultraschallschweißen flüssigkeitsdicht miteinander verschweißt. Alternativ kommen auch Heißsiegeln oder Thermoboden in Frage.

Der Einlass-Vertikalkanal 60 in der ersten Pumpenkammer 41 bildet den Pumpeneinlass und der Auslass-Vertikalkanal 65 der dritten Pumpenkammer 43 bildet den Pumpenauslass.

Durch die Aktuatorkanäle 51,52,53 kann durch entsprechende Schaltung des zugeordneten Umschaltventils 34 pneumatischer Überdruck auf die proximale Rückseite der Pumpenmembrane 46 aufgebracht werden, so dass der Inhalt der betreffenden Pumpenkammer 41,42,43 entleert wird. Durch Umschalten des Umschaltventils 34 kann Unterdruck aus dem Unterdruckspeicher 32 auf die proximale Rückseite der Pumpenmembrane 46 aufgebracht werden, so dass diese in ihre in der Figur 2 dargestellte Ausgangsposition zurückgezogen wird, wodurch die betreffende Pumpenkammer 41,42,43 gefüllt wird.

Durch Füllen der ersten Pumpenkammer 41, anschließendes Füllen der zweiten Pumpenkammer 42 bei gleichzeitigem Leeren der ersten Pumpenkammer 41 und anschließendes Füllen der dritten Pumpenkammer 43 bei gleichzeitigem Leeren der zweiten Pumpenkammer 42 wird ein peristaltisches Pumpen der Flüssigkeit von dem Pumpeneinlass zu dem Pumpenauslass realisiert.

Der absolute Druck in dem Überdruckspeicher 30 liegt ungefähr bei 2,0 bar und der absolute Druck in dem Unterdruckspeicher 32 ungefähr bei 0,5 bar.

Der größte Durchmesser der Pumpkammern 41 -43 beträgt 1,0 bis 5,0 mm, die vertikale Höhe beträgt 0,1 bis 2,0 mm, so dass die Pumpkammern 41-43 ein Volumen von jeweils ungefähr 1,0 bis 10 µl haben.

## Patentansprüche

1. Pneumatisch betätigte Mehrkammer-Peristaltikpumpe (18) für ein Wasseranalysegerät (10), umfassend:
eine Grundplatte (40) mit mehreren proximal offenen Pumpenkammern (41-43) auf der proximalen Seite der Grundplatte (40), mindestens eine die Pumpenkammern (41-43) verschließende, flüssigkeitsdichte und elastische Pumpenmembran (46) auf der proximalen Seite der Grundplatte (40), und
eine Deckplatte (48) auf der Pumpenmembran (46), wobei die Deckplatte (48) im Bereich der Pumpenkammern (41-43) jeweils einen pneumatischen Aktuatorkanal (51-53) aufweist, der zur Betätigung der mindestens einen Pumpenmembran (46) an eine Überdruckquelle (30) anschließbar ist, und
einen Verbindungskanal (71,73) zwischen zwei Pumpenkammern (41,42;42,43), der auf der distalen Seite der Grundplatte (40) als Nut (70,72) ausgebildet ist, wobei
auf der distalen Seite der Grundplatte (40) im Bereich der Verbindungskanal-Nut (70,72) eine separate Nutabdeckung (80) vorgesehen ist, die die distale Öffnungsseite der Verbindungskanal-Nut (70,72) verschließt, **dadurch gekennzeichnet, dass** die Nutabdeckung (80) eine biegsame Abdeckfolie ist, welche sich an Unebenheiten auf der distalen Seite der Grundplatte (40) anpassen kann.

2. Pneumatisch betätigte Mehrkammer-Peristaltikpumpe (18) für ein (10) nach Anspruch 1, wobei die Grundplatte (40) ein Kunststoff-Spritzgussteil ist.

3. Pneumatisch betätigte Mehrkammer-Peristaltikpumpe (18) für ein Wasseranalysegerät (10) nach Anspruch 1 oder 2, wobei die Abdeckfolie (80) in Bezug auf Laserstrahlung transparent ist, die zum Verschweißen der Abdeckfolie (80) mit der Grundplatte (40) verwendet werden kann.

4. Pneumatisch betätigte Mehrkammer-Peristaltikpumpe (18) für ein Wasseranalysegerät (10) nach einem der vorangegangenen Ansprüche, wobei die Grundplatte (40) und die Abdeckfolie (80) aus dem gleichen Kunststoff bestehen und die gleiche Schmelztemperatur aufweisen.

5. Pneumatisch betätigte Mehrkammer-Peristaltikpumpe (18) für ein Wasseranalysegerät (10) nach einem der vorangegangenen Ansprüche, wobei zwischen dem Verbindungskanal (71,73) und den Pumpkammern (41-43) jeweils Vertikalkanäle (61-64) vorgesehen sind, die senkrecht zu dem Verbindungskanal (71,73) stehen.

6. Pneumatisch betätigte Mehrkammer-Peristaltikpumpe (18) für ein Wasseranalysegerät (10) nach einem der vorangegangenen Ansprüche, wobei die Peristaltikpumpe (18) eine mikrofluidische Peristaltikpumpe (18) ist und das Pumpvolumen einer Pumpkammer (41 -43) kleiner als 10 µl ist.

7. Pneumatisch betätigte Mehrkammer-Peristaltikpumpe (18) für ein Wasseranalysegerät (10) nach einem der vorangegangenen Ansprüche, wobei das Wasseranalysegerät (10) aus einem alle elektrischen Bauteile aufweisenden Basismodul (12) und einem austauschbaren Kartuschenmodul (14) besteht, das die gesamte Fluidik einschließlich der Peristaltikpumpe (18) aufweist.

## Claims

1. A pneumatically actuated multi-chamber peristaltic pump (18) for a water analysis device (10), which pump comprises:
a base plate (40) with a plurality of proximally open pump chambers (41-43) on the proximal side of the base plate (40),
at least one liquid-tight and elastic pump membrane (46) on the proximal side of the base plate (40), said membrane closing the pump chambers (41-43), and
a cover plate (48) on said pump membrane (46), said cover plate (48) comprising a respective pneumatic actuator channel (51-53) in the region of the pump chambers (41-43), which is adapted to be connected to an overpressure source for the purpose of actuating the at least one pump membrane (46), and
a connection channel (71, 73) between two pump chambers (41, 42; 42, 43), said channel being formed as a groove (70, 72) on the distal side of the base plate (40), a separate groove cover (80) being provided on the distal side of the base plate (40) in the region of the connection channel groove (70, 72), which cover closes the distal opening side of the connection channel groove (70, 72),
**characterized in that**
the groove cover (80) is a flexible cover film adapted to adjust to unevennesses on the distal side of the base plate (40).

2. The pneumatically actuated multi-chamber peristaltic pump (18) for a water analysis device (10) of claim 1, wherein the base plate (40) is an injection-molded plastic part.

3. The pneumatically actuated multi-chamber peristaltic pump (18) for a water analysis device (10) of claim 1 or 2, wherein the cover film (80) is transparent to laser radiation which may be used to weld the cover film (80) to the base plate (40).

4. The pneumatically actuated multi-chamber peristaltic pump (18) for a water analysis device (10) of one of the preceding claims, wherein the base plate (40) and the cover film (80) are made of the same plastic material and have the same melting temperature.

5. The pneumatically actuated multi-chamber peristaltic pump (18) for a water analysis device (10) of one of the preceding claims, wherein respective vertical channels (61-64) are provided between the connection channel (71, 73) and the pump chambers (41-43), said vertical channels being perpendicular to the connection channel (71, 73).

6. The pneumatically actuated multi-chamber peristaltic pump (18) for a water analysis device (10) of one of the preceding claims, wherein the peristaltic pump (18) is a microfluidic peristaltic pump (18) and the pumping volume of a pump chamber (41-43) is smaller than 10 µl.

7. The pneumatically actuated multi-chamber peristaltic pump (18) for a water analysis device (10) of one of the preceding claims, wherein the water analysis device (10) is formed by a base module comprising all electric components and by an exchangeable cartridge module (14) comprising the entire fluidic system including the peristaltic pump (18).

## Revendications

1. Pompe péristaltique (18) à multiples chambres actionnée pneumatiquement pour un dispositif d'analyse d'eau (10), comprenant:
une plaque de base (40) avec plusieurs chambres de pompe (41-43) sur le côté proximal de la plaque de base (40), les chambres étant ouvertes dans la direction proximale,
au moins une membrane de pompe (46) étanche à liquide et élastique, disposée sur le côté proximal de la plaque de base (40), la membrane fermant les chambres de pompe (41-43), et
une plaque de recouvrement (48) sur la membrane de pompe (46), la plaque de recouvrement (48) comprenant un conduit actionneur (51-53) pneumatique respectif dans la région des chambres de pompe (41-43), qui peut être relié à une source de surpression (30) pour actionner ladite au moins une membrane de pompe (46), et
un conduit de liaison (71, 73) entre deux chambres de pompe (41, 42; 42, 43), qui est formé en forme d'une rainure (70, 72) sur le côté distal de la plaque de base (40),
où, un couvercle de rainure (80) est prévue sur le côté distal de la plaque de base (40) dans la région de la rainure (70, 72) du conduit de liaison, le couvercle fermant le côté ouvert distal de la rainure (70, 72) du conduit de liaison,
**caractérisée en ce que**
le couvercle de rainure (80) est un film de recouvrement flexible apte de s'adapter à des irrégularités sur le côté distal de la plaque de base (40).

2. Pompe péristaltique (18) à multiples chambres actionnée pneumatiquement pour un dispositif d'analyse d'eau (10) selon la revendication 1, dans laquelle la plaque de base (40) est une pièce moulée par injection de matière plastique.

3. Pompe péristaltique (18) à multiples chambres actionnée pneumatiquement pour un dispositif d'analyse d'eau (10) selon les revendications 1 ou 2, dans laquelle le film de recouvrement (80) est transparent au rayonnement laser pouvant être utilisé pour souder le film de recouvrement (80) à la plaque de base (40).

4. Pompe péristaltique (18) à multiples chambres actionnée pneumatiquement pour un dispositif d'analyse d'eau (10) selon l'une quelconque des revendications précédentes, dans laquelle la plaque de base (40) et le film de recouvrement (80) sont formés de la même matière plastique et présentent la même température de fusion.

5. Pompe péristaltique (18) à multiples chambres actionnée pneumatiquement pour un dispositif d'analyse d'eau (10) selon l'une quelconque des revendications précédentes, dans laquelle des conduits verticaux (61-64) sont respectivement prévus entre le conduit de liaison (71, 73) et les chambres de pompe (41-43), les conduits verticaux étant perpendiculaire par rapport au conduit de liaison (71, 73).

6. Pompe péristaltique (18) à multiples chambres actionnée pneumatiquement pour un dispositif d'analyse d'eau (10) selon l'une quelconque des revendications précédentes, dans laquelle la pompe péristaltique (18) est une pompe péristaltique (18) microfluidique et le volume de pompage d'une chambre de pompe (41-43) est inférieure à 10 µl.

7. Pompe péristaltique (18) à multiples chambres actionnée pneumatiquement pour un dispositif d'analyse d'eau (10) selon l'une quelconque des revendications précédentes, dans laquelle le dispositif d'analyse d'eau (10) est formé par un module de base (12) comprenant tous les composants électriques et par un module de cartouche (14) échangeable comportant tout le système fluidique incluant la pompe péristaltique (18).
